# EUROPEAN PATENT SPECIFICATION

(11) **EP 1 703 902 B1**
(45) Date of publication and mention of the grant of the patent: **03.11.2010**
(21) Application number: 05711256.7
(22) Date of filing: 05.01.2005
(51) Int. Cl.: A61K 31/13, A61K 45/06, A61P 25/28

(54) **MEMANTINE FOR THE TREATMENT OF MILD-TO-MODERATE ALZHEIMER'S DISEASE**
MEMANTIN FÜR DIE BEHANDLUNG VON LEICHTEM BIS MITTELSCHWEREM MORBUS ALZHEIMER
MEMANTINE DESTINEE AU TRAITEMENT DE LA MALADIE D'ALZHEIMER LEGERE A MODEREE

(30) Priority: 05.01.2004 US 534553 P; 04.02.2004 US 542176 P
(43) Date of publication of application: 27.09.2006
(73) Proprietor: Merz Pharma GmbH & Co. KGaA, 60318 Frankfurt (DE)
(72) Inventor: McDONALD, Scott, Chathem, NJ 07928 (US); GERGEL, Ivan, Malvern, PA 19355 (US); STÖFFLER, Albrecht, D-60487 Frankfurt am Main (DE); MOEBIUS, Hans-Joerg, 60322 Frankfurt am Main (DE); WIRTH, Yvonne, D-35041 Marburg (DE); POTKIN, Steven, Irvine, CA 92617-4045 (US)
(74) Representative: Goddard, Christopher Robert
(86) International application number: PCT/US2005/000145
(87) International publication number: WO 2005/067908

(56) References cited:
- WO-A-03/101458
- WO-A-2004/071431
- US-A1- 2004 102 525
- GÖRTELMEYER R ET AL: "Memantine in the treatment of mild to moderate dementia syndrome. A double-blind placebo-controlled study." ARZNEIMITTEL-FORSCHUNG. JUL 1992, vol. 42, no. 7, July 1992 (1992-07), pages 904-913, XP001006077 ISSN: 0004-4172 cited in the application
- DATABASE MEDLINE [Online] US NATIONAL LIBRARY OF MEDICINE (NLM), BETHESDA, MD, US; 2000, ANDROSOVA L V ET AL: "[Akatinol memantin in Alzheimer's disease: clinico-immunological correlates]" XP002333419 Database accession no. NLM11026134 & ZHURNAL NEVROLOGII I PSIKHIATRII IMENI S.S. KORSAKOVA / MINISTERSTVO ZDRAVOOKHRANENIIA I MEDITSINSKOI PROMYSHLENNOSTI ROSSIISKOI FEDERATSII, VSEROSSIISKOE OBSHCHESTVO NEVROLOGOV [I] VSEROSSIISKOE OBSHCHESTVO PSIKHIATROV. 2000, vol. 100, no. 9, 2000, pages 36-38,
- PANTEV M., RITTER R., GÖRTELMEYER R.: "Clinical and behavioural evaluation in long-term care patients with mild to moderate dementia under Memantine treatment" ZEITSCHRIFT FÜR GERONTOPSYCHOLOGIE UND -PSYCHIATRIE, vol. 6, no. 2, 1993, pages 103-117, XP008048669
- WILCOCK G ET AL: "A double-blind, placebo-controlled multicentre study of memantine in mild to moderate vascular dementia (MMM500)." INTERNATIONAL CLINICAL PSYCHOPHARMACOLOGY, vol. 17, no. 6, November 2002 (2002-11), pages 297-305, XP008048656 ISSN: 0268-1315
- "Non-cholinergic strategies have a potential role in the management of Alzheimer's disease" DRUGS AND THERAPY PERSPECTIVES 2003 NEW ZEALAND, vol. 19, no. 10, 2003, pages 7-10, XP008048701 ISSN: 1172-0360
- ROGAWSKI M A: "What is the rationale for new treatment strategies in Alzheimer's disease?" CNS SPECTRUMS 2004 UNITED STATES, vol. 9, no. 7 SUPPL. 5, 2004, pages 6-12+31, XP008048660 ISSN: 1092-8529
- FERET B. ET AL: "Memantine : An oral NMDA antagonist for the treatment of moderate-to-severe Alzheimer's disease." FORMULARY, VOL. 39, NO. 2, PP. 91-103. REFS: 20 ISSN: 1082-801X CODEN: FORMF, 2004, XP008048658
- SCHMITT F A (REPRINT) ET AL: "Efficacy of Memantine for cognitive deficits of mild to severe Alzheimer 's disease" NEUROPSYCHOPHARMACOLOGY, (DEC 2004) VOL. 29, SUPP. [1], PP. S131-S131. PUBLISHER: NATURE PUBLISHING GROUP, MACMILLAN BUILDING, 4 CRINAN ST, LONDON N1 9XW, ENGLAND. ISSN: 0893-133X., December 2004 (2004-12), XP008048661
- POMARA N ET AL: "O1-05-04 Memantine monotherapy is effective and safe for the treatment of mild to moderate Alzheimer's disease: a randomized controlled trial" NEUROBIOLOGY OF AGING, TARRYTOWN, NY, US, vol. 25, July 2004 (2004-07), page S19, XP004624677 ISSN: 0197-4580
- TARIOT P N ET AL: "P1-021 Memantine treatment for mild to severe Alzheimer's disease: clinical trials summary" NEUROBIOLOGY OF AGING, TARRYTOWN, NY, US, vol. 25, July 2004 (2004-07), pages S99-S100, XP004624949 ISSN: 0197-4580
- POTKIN S G ET AL: "P3-102 Memantine monotherapy increases brain metabolism (PET) and effectively treats mild to moderate Alzheimer's disease" NEUROBIOLOGY OF AGING, TARRYTOWN, NY, US, vol. 25, July 2004 (2004-07), page S384, XP004625868 ISSN: 0197-4580
- OLSEN C.E. ET AL: "Drug therapy of dementia in elderly patients. A review." NORDIC JOURNAL OF PSYCHIATRY, VOL. 59, NO. 2, PP. 71-77. REFS: 40 ISSN: 0803-9488 CODEN: NJPYEJ, 2005, XP008048700

## Description

### FIELD OF THE INVENTION

The present invention relates to use of memantine or a pharmaceutically acceptable salt thereof for the manufacture of a medicament for monotherapeutically treating as specified in the claims.

### BACKGROUND OF THE INVENTION

### Dementia and Alzheimer's Disease

Dementia is a serious disorder affecting as many as 10% of individuals older than 65 years and more than 24% of those older than 85 years (Hofman et al., Int. J. Epidemiol. 1991; 20:736-748; Jorm and Jolley, Neurology, 1998; 51:728-733; Lobo et al., Neurology. 2000; 54(Suppl. 5):S4-S9). Alzheimer's disease (AD) is an increasingly prevalent form of neurodegeneration that accounts for approximately 50% - 60 % of the overall cases of dementia among people over 65 years of age. Dementia associated with AD is referred to as senile dementia of the Alzheimer's type (SDAT), in common usage with Alzheimer's disease. AD is characterized clinically by progressive loss of memory, cognition, reasoning, judgment, and emotional stability that gradually leads to profound mental deterioration and ultimately, death. AD is a progressive disorder with a mean duration of around 8.5 years between onset of clinical symptoms and death. AD is believed to represent the fourth most common medical cause of death and affects about 4-5 million people in the United States. Prevalence of AD doubles every 5 years beyond age 65 (National Institute on Aging: Prevalence and costs of Alzheimer's disease. Progress Report on Alzheimer's Disease. NIH Publication No. 99 3616, November 1998; Polvikoski et al., Neurology. 2001; 56:1690-1696). AD currently affects about 15 million people world-wide (including all races and ethnic groups) and owing to the relative increase of elderly people in the population its prevalence is likely to increase over the next two to three decades. AD is at present incurable. No treatment that effectively prevents AD or reverses its symptoms and course is currently known.

Neuronal death. AD is associated with death of pyramidal neurons and loss of neuronal synapses in brain regions associated with higher mental functions (Francis et al., J. Neurol. Neurosurg. Psychiatry. 1999; 66:137-147). The brains of individuals with AD exhibit characteristic lesions termed senile (or amyloid) plaques, amyloid angiopathy (amyloid deposits in blood vessels) and neurofibrillary tangles. Smaller numbers of these lesions in a more restricted anatomical distribution are also found in the brains of most aged humans who do not have clinical AD. Amyloid plaques and amyloid angiopathy also characterize the brains of individuals with Trisomy 21 (Down's Syndrome) and Hereditary Cerebral Hemorrhage with Amyloidosis of the Dutch-Type (HCHWA-D). At present, a definitive diagnosis of AD usually requires observing the aforementioned lesions in the brain tissue of patients who have died with the disease or, rarely, in small biopsied samples of brain tissue taken during an invasive neurosurgical procedure. Nevertheless, physicians routinely diagnose AD based on a battery of symptoms, relying on the known correlation between such symptoms and the symptomology obtained on biopsy.

AD is associated with a profound loss of cholinergic neurons within the nucleus basalis of Meynert (Perry et al., Br. Med. J. 1978; 2:1456-1459; Geula and Mesulam, Cholinergic systems and related neuropathological predilection patterns in Alzheimer disease. In: Alzheimer's Disease. Terry et al. eds.; New York: Raven Press; 1994, pp. 263-291). The signaling in these neurons is mediated by the extracellularly released neurotransmitter acetylcholine (ACh). Drugs that selectively enhance cholinergic function by inhibition of the cholinergic catabolic enzyme acetylcholinesterase (AChE), which destroys ACh after the latter has been secreted into the synaptic clefts (Goff and Coyle, Am. J. Psychiatry. 2001; 158: 1367-1377) have been used to treat AD. At present, the most widely clinically used acetylcholinesterase inhibitors (AChEI) are donepezil, galantamine, and rivastigmine.

Dementia and metabolism. Structural neuroimaging has an important role in initial evaluation of dementia for ruling out potentially treatable causes. Dementia associated with AD is distinguished from other forms of dementia by a characteristic bilateral pattern of decreased metabolism that is most prominent in temporal, parietal and prefrontal associational areas, and also in the posterior cingulate and precuneus, with relative sparing of primary sensory and motor cortices, basal ganglia, thalamus, brainstem, and cerebellum (Small & Leiter, J Clin Psychiatry. 1998; 59 Suppl 11: 4-7). The severity of decline in glucose metabolic rate parallels the severity of dementia and correlates with regional neuronal loss (McGeer et al., Can J Neurol Sci. 1986; 13(4 Suppl):511-6). The brain regions most affected by Alzheimer's disease change over time. ¹⁸F-flurodeoxyglucose positron emission tomography (FDG PET) *in vivo* metabolic studies have demonstrated hypometabolism in widespread cortical areas, including frontal and temporal lobe and hippocampal area, beginning in the early stages of AD. FDG PET measures regional blood flow and/or glucose metabolism and is hypothesized to have the capacity to detect functional abnormalities before structural changes appear (Ichimiya et al., Psychiatry Clin Neurosci. 1998; 52 Suppl: S223-5; Reiman et al., Proc Natl Acad Sci U S A. 2001 13; 98(6): 3334-9; Silverman et al., J Nucl Med. 2002; 43(2): 253-66; Small et al., Curr Neurol Neurosci Rep. 2003; 3(5): 385-92).

### NMDA-Receptor Antagonists and Memantine

The excessive or pathological activation of glutamate receptors, particularly those that are selectively activated by N-methyl-D-aspartate (NMDA), has also been implicated in the processes that underlie the degeneration of cholinergic cells in the brains of AD patients (Greenamyre et al., Neurobiol. Aging. 1989; 10:593-602; Francis et al., J. Neurochem. 1993; 60:263-291; Li et al., J. Neuropathol. Exp. Neurol. 1997; 56:901-911; Wu and Rowan, Neuroreport. 1995;,6:2409-2413). The NMDA receptor is pivotal for several physiologic synaptic plasticity processes including memory and learning (Collinridge and Singer, Trends Pharmacol. Sci. 1990; 11: 290-296).

NMDA receptor antagonists potentially have a wide range of therapeutic applications in numerous CNS disorders such as acute neurodegeneration (*e.g*., associated with stroke and trauma), chronic neurodegeneration (*e*.*g*., associated with Parkinson's disease, AD, Huntington's disease, and amyotrophic lateral sclerosis [ALS]), epilepsy, drug dependence, depression, anxiety, and chronic pain (for reviews *see:* Parsons et al., Drug News Perspect. 1998; 11:523-569; Parsons *et al*., 1999, *supra*; Jentsch and Roth, Neuropsychopharmacology. 1999; 20: 201-205; Doble, Therapie. 1995; 50: 319-337). Functional inhibition of NMDA receptors can be achieved through actions at different recognition sites within the NMDA receptor complex. Such sites include i) the primary transmitter site (competitive); ii) the phencyclidine site located inside the cation channel (uncompetitive); iii) the polyamine modulatory site, and iii) the strychnine-insensitive, co-agonistic, allosteric, glycine site (glycine B) (Parsons *et al.,* 1999, *supra*).

It is believed that the excitatory neurotransmitter glutamate plays an important role in the pathophysiology (as opposed to etiology) of neurodegenerative diseases such as AD, Parkinsons's disease, and amyotrophic lateral sclerosis. About 70% of all excitatory synapses in the CNS are stimulated by glutamate, and dysfunctional, chronic release of glutamate can produce a prolonged excitatory effect via glutamate receptor activation. This prolonged activation is mediated by the NMDA glutamate receptor and can result in the degeneration and death of cortical neurons.

Memantine (1-amino-3,5-dimethyl adamantane) is an analog of 1-amino-cyclohexane (disclosed, *e.g*., in U.S. Patent Nos. 4,122,193; 4,273,774; 5,061,703) which is a systemically-active uncompetitive NMDA receptor antagonist having moderate affinity for the receptor, strong voltage dependency and rapid blocking/unblocking kinetics. Memantine and other 1-aminoalkylcyclohexanes have proven useful in alleviation of various progressive neurodegenerative disorders such as dementia in patients with moderate to severe AD, Parkinson's disease, and spasticity (*see, e*.*g*., U. S. Patent Nos. 5,061,703; 5,614,560, and 6,034,134; Parsons *et al*., 1999, *supra*; Möebius, ADAD. 1999; 13:S172-178; Danysz et al., Neurotox. Res. 2000; 2:85-97; Winblad and Poritis, Int. J. Geriatr. Psychiatry. 1999; 14:135-146; Görtelmeyer *et al.* 1992, *supra*; Danysz et al., Curr. Pharm. Des. 2002;,8:835-843; Jirgensons et. al., Eur. J. Med. Chem. 2000; 35: 555-565).

Memantine is currently approved in Europe for the treatment of moderately severe to severe AD, and in the United States for the treatment of moderate to severe AD. In addition, memantine when given to moderate to severe AD patients maintained on the AChEI donepezil resulted in unexpected greater relief of symptoms, compared to AD patients receiving placebo. This effect has not been demonstrated in patients with mild to moderate AD, where combination therapy involving administration of memantine and the AChEIs did not result in any benefit compared with the AChEI alone.

Görtelmeyer et al. disclose in "Memantine in the treatment of mild to moderate dementia syndrome. A double-blind placebo-controlled study.", Arzneimittel-Forschung, 1992, vol. 42, no. 7, pages 904-913, that memantine improves mild to moderate dementia syndrome.

Androsova et al. teach in "Akatinol memantin in Alzheimer's disease: clinico-immunological correlates", Zhurnal nevrologii i psikhiatrii imeni S.S. Korsakova / Ministerstvo zdravookhraneniia i meditsinskoi promyshlennosti Rossiskoi Federatsii, Vserossiiskoe obshchestvo nevrologov [i] Vserossiiskoe obshchestvo psikhiatrov, 2000, vol. 100, no. 9, pages 36-38, that memantine has an influence on IL-1 production in Alzheimer patients with severe, moderate and mild dementia.

The prior right WO 2004/071431 A, discloses that memantine shows efficacy in treating mild-to-moderate Alzheimer's disease.

### SUMMARY OF THE INVENTION

The present invention provides, in an embodiment, the use of memantine or a pharmaceutically acceptable salt thereof in the manufacture of a medicament for monotherapeutically treating mild-to-moderate Alzheimer' s disease, wherein the medicament is manufactured for administration of memantine at an initial dose of 5 mg/day rising weekly by 5 mg/day to a final dose of 20 mg/day.

In another embodiment, the present invention provides the use of memantine or a pharmaceutically acceptable salt thereof in the manufacture of a medicament for monotherapeutically treating mild-to-moderate Alzheimer' s disease, wherein the medicament is manufactured for administration to subjects with mild-to-moderate Alzheimer' s disease who have previously been treated with an acetylcholinesterase inhibitor but have discontinued said previous treatment at least one day prior to commencement of memantine administration.

In one embodiment, memantine is administered in a range from about 5-100 mg/day.

In another embodiment, memantine is administered in a range from about 10-40 mg/day.

In yet another embodiment, memantine is administered at a dose of about 20 mg/day.

### BRIEF DESCRIPTION OF THE DRAWINGS

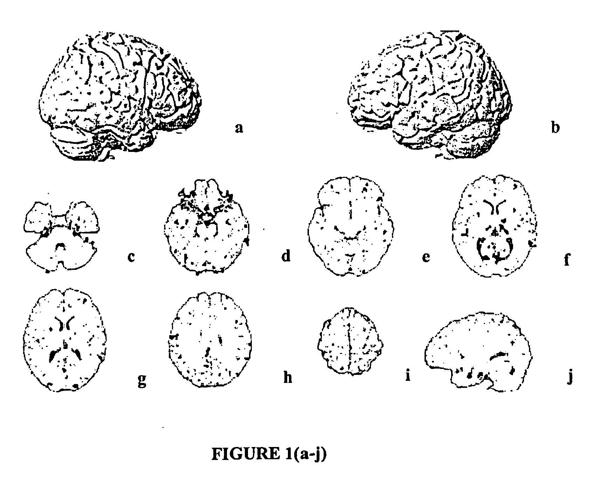

Figure 1. Figure 1 demonstrates differences in regional brain metabolic activity assessed by PET in mild-to-moderate AD subjects following 24 weeks of placebo compared to baseline. Mean levels of relative metabolic activity are superimposed on a standard surface rendering template from SPM. Red indicates higher levels of metabolic activity following placebo; blue indicates lower levels of activity. Statistically significant (p < .025) regional differences are determined with a 10-voxel extent threshold to adjust for multiple comparisons. Images a and b represent the lateral cortical surfaces; images c through i are axial images (z = -31mm, -19mm, -8mm, 8mm, 14mm, 29mm, 60mm relative to the AC-PC line, respectively); image j is a sagittal image at +33mm (right side of brain). The x, y, and z coordinates correspond to Talairach space.

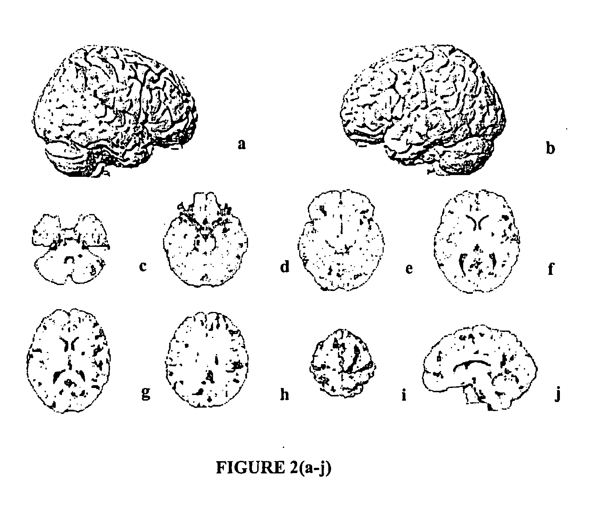

Figure 2. Figure 2 demonstrates differences in regional brain metabolic activity assessed by PET in AD subjects following 24 weeks of 20 mg memantine per day compared to baseline. Red indicates higher levels of metabolic activity following Memantine; blue indicates lower levels of activity. Statistically significant (p < .025) regional differences are determined with a 10-voxel extent threshold to adjust for multiple comparisons. Image descriptions are the same as those given in Figure 1, except that image j is a sagittal image at -4 mm (left side of brain).

### DETAILED DESCRIPTION

The present invention unexpectedly demonstrates that patients with mild-to-moderate senile dementia of the Alzheimer's type (SDAT) benefit from treatment with memantine compared with placebo. This result is surprising in view of a previous study in which addition of memantine to an AChEI administration regimen did not demonstrate improved efficacy compared with AChEI therapy alone for the treatment of mild-to-moderate AD. The present result is unexpected for the further reason that the majority of the patient population treated with memantine were individuals who were previously treated with AChEI but either had discontinued AChEI therapy either because they did not respond, or because they could not tolerate the adverse side effects, or both.

The present invention is based on results from a randomized, double-blind, placebo-controlled, multi-center, parallel-group study comparing memantine to placebo in outpatients diagnosed with probable AD according to NINCDS-ADRDA criteria. Severity of mild to moderate was established using the Mini-Mental State Examination (MMSE) score ( ≥10 and ≤22; 0 is the lowest score and 30 is the highest score).

The present invention also unexpectedly demonstrates that memantine administered to patients with mild-to-moderate AD prevents the reduction of glucose metabolism in the cortical and subcortical regions of such patients relative to placebo. These data are based on results from PET scanning procedures in 10 patients (5 on memantine and 5 on placebo) at one site of the multi-center trial described above.

In the study of the present invention, there were approximately 400 patients enrolled across 42 centers. Evaluation of primary efficacy was achieved by Alzheimer's Disease Assessment Scale-cognitive subscale (ADAS-cog) and the Clinician's Interview-Based Impression of Change including caregiver information (CIBIC-plus). Secondary efficacy was determined using Alzheimer's disease Cooperative Study-Activities of Daily Living (ADCS-ADL) and Neuropsychiatric Inventory (NPI) criteria.

The ADAS-cog comprises an 11-item scale that is used to assess the severity of selected areas of cognitive impairment (memory, language, orientation, reason and praxis). Scores range from 0 to 70 with lower scores indicating lesser severity and a score of 70 representing the worst cognitive impairment. Its use in assessing and following changes in patients with mild to moderate Alzheimer's disease has been extensively validated. The ADAS-cog was administered at each clinic visit starting with the Baseline visit and at the end of weeks 4, 8, 12, 18 and 24 (or upon early termination).

The CIBIC-Plus is a global rating that was derived through an independent, comprehensive interview with the patient and caregiver by an experienced rater/clinician who was barred from knowledge of all other psychometric test scores (after Baseline visit) conducted as part of this protocol and who is not otherwise familiar with the patient. Scores 1-3 indicate improvement; Score 4 indicates no change (as compared to baseline); Scores 5-7 indicate worsening. The CIBIC rater assessed disease severity at Baseline. Using the results from Baseline for reference, the rater then interviewed the patient and caregiver at the end of Weeks 4, 8, 12, 18 and 24 (or upon early termination), to obtain an "Impression of Change" rating. The format for this scale was derived from the Alzheimer's Disease Cooperative Study - Clinician's Global Impression of Change scale (ADCS-CGIC) (Schneider, L. et al, Alzheimer Dis Assoc Disord. 1997; 11 Suppl 2:S22-32). The CIBIC-plus was administered at each clinic visit starting with the Baseline visit.

The ADCS-ADL inventory consists of 23 questions used to measure the functional capabilities of patients with dementia. These questions are selected from a larger set of 49 questions in the original ADL scale. A more common selection is of 19 questions from the same 49 question group. Each ADL item comprises a series of hierarchical sub-questions, ranging from the highest level of independent performance of each ADL to complete loss. The ADSC-ADL Inventory total score ranges from 0 (lower functioning status) to 78 (higher functioning status). A higher score indicates a better functioning status. The inventory is performed by interviewing a person who is in close contact with the patient and covers the most usual and consistent performance of the patient over the preceding four weeks (Galasko et al., Alzheimer Dis Assoc Disord. 1997; 11 Suppl 2:S33-9). The ADCS-ADL was administered at each clinic visit starting with the Baseline visit.

The NPI is a validated scale that assesses behavioral disturbances in patients with dementia (Cummings et al., Neurology. 1994; 44(12):2308-14). It provides both a total score (sum of 12 domain scores) as well as scores for each subscales (*e.g*., delusions, hallucinations, agitation/aggression, depression/dysphoria, anxiety, disinhibition, elation/euphoria, apathy/indifference, irritability/lability, aberrant motor activity). For each subscale, both the frequency and the severity of each behavior is measured. The NPI total score ranges from 0 (higher functioning status) to 144 (lower functioning status). The NPI is based upon responses from the caregiver. The NPI was administered at Baseline and the end of Weeks 12 and 24 (or upon early termination).

After 24 weeks, statistically significant improvement was shown according to the ADAS-cog, CIBIC-plus, and NPI overall criteria.

### Definitions

"Memantine" refers to 1-amino-3,5-dimethyladamantane or pharmaceutically acceptable salts, such as the hydrochloride salt. In the United States, the trade name for memantine is Namenda®, in Germany Akatinol and in the European Union Auxura, and Ebixa. Memantine is the subject matter of U.S. Patent Nos. 4,122,193 and 4,273,774.

Various salts and isomers (including stereoisomers and enantiomers) of memantine can be used. The term "salts" can include acid addition salts or addition salts of free bases. Examples of acids which may be employed to form pharmaceutically acceptable acid addition salts include inorganic acids such as hydrochloric, sulfuric, or phosphoric acid, and organic acids such as acetic, maleic, succinic, or citric acid. All of these salts (or other similar salts) may be prepared by conventional means. The nature of the salt is not critical, provided that it is non-toxic and does not substantially interfere with the desired pharmacological activity. A preferred salt for the present invention is the hydrochloride salt.

The phrase "pharmaceutically acceptable", as used in connection with compositions of the invention, refers to molecular entities and other ingredients of such compositions that are physiologically tolerable and do not typically produce untoward reactions (toxicity or side effects) when administered to a mammal (*e.g*., human). Preferably, as used herein, the term "pharmaceutically acceptable" means approved by a regulatory agency of the Federal or a state government or listed in the U.S. Pharmacopeia or other generally recognized pharmacopeia for use in mammals, and more particularly in humans.

The term "carrier" applied to pharmaceutical compositions of the invention refers to a diluent, excipient, or vehicle with which an active compound (*e.g*., an 1-aminocyclohexane derivative) is administered. Such pharmaceutical carriers can be sterile liquids, such as water, saline solutions, aqueous dextrose solutions, aqueous glycerol solutions, and oils, including those of petroleum, animal, vegetable or synthetic origin, such as peanut oil, soybean oil, mineral oil and, sesame oil. Suitable pharmaceutical carriers are described in "Remington's Pharmaceutical Sciences" by E.W. Martin, 18th Edition.

The term "patient" or "subject in need thereof" as used herein refers to a mammal. In particular, the term refers to humans diagnosed with mild-to-moderate AD. In particular, the term refers to humans diagnosed with mild-to-moderate AD who also demonstrate reductions in glucose metabolic rate (GMR) compared to individuals without AD.

"Mild-to-moderate senile dementia of the Alzheimer's type" (SDAT), or mild-to-moderate Alzheimer's can be diagnostically assessed as "probable Alzheimer's" according to the National Institute of Neurological and Communicative Disorders and Stroke/the Alzheimer's Disease and Related Disorders Associations (NINCDS-ADRDA) criteria, described above, and in the Examples below. The NINCDS-ADRDA criteria for probable Alzheimer's are provided in Example I.

The diagnosis of "mild-to-moderate" is well within the purview of the ordinary skilled physician using standard criteria, including the clinical assessment scales disclosed above and below. By way of example, the following numerical ranges on the standardized Mini-Mental State Examination (MMSE; 0-30 scale) have been used to diagnose mild-to-moderate, moderate, and moderate-to-severe Alzheimer's.

Mild-to-moderate AD has been diagnosed as determined by MMSE scores of 10 to 22 in the present study, and also from 10-26 in studies using other therapeutics for treating mild-to-moderate Alzheimer's (*e.g*., donepezil). Moderate-to-severe Alzheimer's has been diagnosed in subjects having MMSE scores from 3 to 14, or 5 to 14.

Accordingly, a diagnosis of "mild" Alzheimer's disease could be made for subjects having the higher scores within the above-described ranges, *e.g*., about 15 to 26 on the MMSE, preferably about 15-22. Mild Alzheimer's also has been diagnosed within an MMSE range of 18-22.

The above is contrasted with severe Alzheimer's disease, which has been determined by scores of 5 to 9 on the MMSE. Severe Alzheimer's has also been diagnosed when the MMSE score was less than 10.

It should be noted that the MMSE scale is not the only way to diagnose mild Alzheimer's disease, but represents a convenience. Nor should the claims be construed as requiring the step of "grading" a patient on the MMSE scale to be performed. Thus a patient having mild Alzheimer's disease is a patient who would score 15 or higher if the patient were scored according to MMSE scale. If a different scale were to be used, "mild" AD would be defined as a diagnosis of AD or probable AD which is made based on a score that clearly does not overlap with the score range for moderate-to-severe AD established for the same scale.

The term "treat" is used herein to mean to relieve or alleviate at least one symptom of a disease in a subject. For example, in relation to dementia, the term "treat" may mean to relieve or alleviate cognitive impairment (such as impairment of memory and/or orientation) or impairment of global functioning (activities of daily living) and/or slow down or reverse the progressive deterioration in ADL or cognitive impairment in individuals having mild-to-moderate SDAT.

Within the meaning of the present invention, the term "treat" also denotes inhibition, *i*.*e*., prevention of or delay of the progression of, disease in the patients presenting with additional symptoms associated with SDAT, such as those identified using one or more of the ADAS-cog and CIBIC-plus criteria, the ADCS-ADL criteria, or the NPI total criteria, defined above. The term "delay the progression" is used herein to mean slower than expected development or continuance or aggravation of a disease in a subject compared to an untreated subject. This can be determined for Alzheimer's disease, for example, by obtaining slower than expected deterioration in measures such as cognitive performance in treated patients, compared with those measures in untreated patients (who represent the expected progression of the disease). Cognitive performance can be measured using, *e.g*., the Alzheimer's Disease Assessment Scale (ADAS-cog), or the Alzheimer's Disease Cooperative Study-Activities of Daily Living (ADCS-ADL). For example, the typical disease progression in subjects with mild Alzheimer's disease is an increase of about 1 to about 3 points on the ADAS-cog over a time period of about 6 months. However, disease progression is highly individualized, and also depends on factors such as the initial condition of the patient. Thus, the expected slope of deterioration will be "flatter" for a patient who is in good physical condition and has only mild impairment than that of a patient who is in poorer physical condition and has more pronounced impairment, even if both patients are scored as suffering from "mild" Alzheimer's disease. This is appreciated by those skilled in the art.

The term "treat" also means to increase the glucose metabolic rate, or to inhibit further reduction in the metabolic rate in the cortical and sub-cortical regions of brains in patients with mild-to-moderate AD, which is associated with regression. This can also be assessed by comparing the glucose metabolism in treated patients with that in untreated patients. A reduction in the decrease of glucose metablolism in the treated patients, or a slower than expected decrease, or stability of glucose metabolism in treated patients, compared with untreated patients, is indicative of a benefit accompanying the treatment. This can be determined, *e.g*., by positron emission tomography (PET) scans, or as calculated, *e.g*., by superimposition on a standard surface template and statistically determined using statistical parametric mapping. However, any known technique in the pertinent art can be used to assess changes in glucose metabolism, including high-resolution MRI.

The term "prophylactically treat" also means prophylactic use of memantine in a subject to avert behavior or events associated with SDAT in mild-to-moderate AD. Subjects having or at risk for developing AD, such as those with a familial pattern of AD, can be identified by diagnostic or prognostic assays according to the ordinary skill in the art. In a specific embodiment, the term "prophylactically treat" refers to inhibition (i.e., prevention , delay or alleviation) of the reduction in glucose metabolism in the cortical and sub-cortical regions of brains in individuals with mild-to-moderate AD or individuals who present with this symptom without otherwise fulfilling the criteria for a definitive or even tentative diagnosis of AD.

The term "therapeutically effective amount" is used herein to mean an amount or dose of memantine that is effective to ameliorate, delay, or prevent a symptom, behavior or event associated with SDAT in patients with mild-to-moderate AD. Alternatively, a therapeutically effective amount is sufficient to cause an improvement in a clinically significant condition or parameter (according to the attending physician employing one or more of the foregoing sets of criteria) associated with SDAT in an individual in need thereof.

A "responder" is defined as a patient for whom the change from baseline (Visit 2) to Week 24 in ADAS-cog is less than or equal to - 4. A responder in terms of CIBIC-plus ' is defined as a patient for whom CIBIC-plus equals to "Markedly improved", or "Moderately improved", or "Minimally improved", or "no change" at Week 24.t

An "adverse event" (AE) is determined according to the World Health Organization (WHOART, Version 1998/Q4) Dictionary. An AE occurring during the double-blind treatment period was considered a treatment emergent AE (TEAE) if it was not present prior to the start of double-blind study medication, or if it was present prior to the start of double-blind study medication but increased in severity during the double-blind treatment period. An AE which occurred more than 30 days after the date of the last double-blind study medication (AE start date - the last double-blind study medication date > 30), was not counted as a TEAE. A "serious adverse event" (SAE) is one that results in death, is an immediate threat to life, requires inpatient hospitalization, or results in persistent or significant disability/incapacity.

The "cortical and subcortical" regions of the brain include but are not limited to the orbital cortex; posterior cingulated; retrosplenial and posterior parahippocampal gyrus; superior temporal gyrus; visual cortices; dorsolateral prefrontal cortex; posterior thalamus; cerebellar cortex; right temporal pole and middle temporal gyrus; right medial thalamus; left putamen; right superior parietal and right premotor cortex, left motor cortex; right insula; limbic structures such as the lateral amygdale, orbital cortex; the border between the parahippocampal gyrus and medial fusiform gyrus; anterior temporal lobe; anterior and posterior attentional system; the premotor, inferior parietal (language-related) temporal tip; and orbital and dorsal prefrontal cortices.

The terms "bilateral" or "bilaterally" refer to both sides of the brain being included, or regions thereof, while the term "unilateral" or "unilaterally" refer to only one side of the brain..

"FDG PET" refers to a computerized image used to quantify changes in metabolic activity of body tissues.

The "glucose metabolic rate (GMR)" refers to how rapidly the brain metabolizes glucose. The brain represents approximately 2% of the body's total weight yet accounts for 20% of the body's total oxygen utilization and 65%, of its glucose needs (Lundgren (ed.) Acute neuroscience nursing: concepts and care. Boston: Jones & Bartlett, 1986). Different brain structures have different metabolic needs. The gray matter of the brain, particularly the frontal lobes, has the highest metabolic requirements. The metabolic rates of the occipital, parietal, and temporal lobes along with the basal ganglia and cerebellum are lower. The brainstem has the lowest metabolic need.

The terms "about" and "approximately" shall generally mean an acceptable degree of error or variation for the quantity measured given the nature or precision of the measurements. Typically, degrees of error or variation are within 20 percent (%), preferably within 10%, and more preferably within 5% of a given value or range of values. Numerical quantities given herein are approximate unless stated otherwise, meaning that the term "about" or "approximately" can be inferred when not expressly stated.

### Formulation, Dosage, and Administration

Memantine (NAMENDA®) is commercially available as the hydrochloride salt. According to the present invention, the dosage form of memantine may be a solid, semisolid or liquid formulation. Formulation of memantine in semi-solid or liquid form is within the skill of the art, as the active ingredient is highly soluble in aqueous media. Usually the active substance, i.e., memantine, will constitute between 0.1 and 99% by weight of the formulation, more specifically between 0.5 and 20% by weight for formulations intended for injection and between 0.2 and 50% by weight for formulations suitable for oral administration.

The pharmaceutical formulation comprises the active ingredients, optionally in association with pharmaceutically acceptable adjuvants, diluents, excipients and/or inert carriers.

To produce pharmaceutical formulations of the combination of the invention in the form of dosage units for oral application, the selected compounds may be mixed with a solid excipient, *e.g*., lactose, saccharose, sorbitol, mannitol, starches such as potato starch, corn starch or amylopectin, cellulose derivatives, a binder such as gelatine or polyvinylpyrrolidone, disintegrants, *e.g*., sodium starch glycolate, cross- linked PVP, cross-carmellose sodium and a lubricant such as magnesium stearate, calcium stearate, polyethylene glycol, waxes, paraffin, and the like, and then compressed into tablets. If coated tablets are required, the cores, prepared as described above, may be coated with a concentrated sugar solution which may contain *e.g*., gum arabic, gelatine, talcum, titanium dioxide, and the like. Alternatively, the tablets can be coated with a polymer known to the man skilled in the art, wherein the polymer is dissolved in a readily volatile organic solvent or mixture of organic solvents. Dyestuffs may be added to these coatings in order to readily distinguish between tablets containing different active substances or different amounts of the active compounds.

For the formulation of soft gelatin capsules, the active substances may be admixed with *e.g*., a vegetable oil or poly-ethylene glycol. Hard gelatin capsules may contain granules of the active substances using either the above mentioned excipients for tablets *e.g*., lactose, saccharose, sorbitol, mannitol, starches (*e.g*., potato starch, corn starch or amylopectin), cellulose derivatives or gelatin. Also liquids or semisolids of the drug can be filled into hard gelatin capsules.

Unit dosage formulations for oral administration may be provided in *e.g*., blister packs. Memantine tablets are available in blister packages of 50 or 100 tablets.

Dosage units for rectal application can be solutions or suspensions or can be prepared in the form of suppositories comprising the active substances in a mixture with a neutral fatty base, or gelatin rectal capsules comprising the active substances in admixture with vegetable oil or paraffin oil. Liquid formulations for oral application may be in the form of syrups or suspensions, for example solutions containing from about 0.2% to about 20% by weight of the active substances herein described, the balance being sugar and mixture of ethanol, water, glycerol and propylene glycol. Optionally such liquid formulations may contain coloring agents, flavoring agents, saccharine and carboxymethyl-cellulose as a thickening agent or other excipients known to a person skilled in the art.

Solutions for parenteral applications by injection can be prepared in an aqueous solution of a water-soluble pharmaceutically acceptable salt of the active substances, preferably in a concentration of from about 0.5% to about 10% by weight. These solutions may also contain stabilizing agents and/or buffering agents and may conveniently be provided in various dosage unit ampoules.

Suitable daily doses of the memantine for the therapeutic treatment of humans are about 0.01-10 mg/kg bodyweight on peroral administration and 0.001-10 mg/kg bodyweight on parenteral administration.

In one embodiment, 5 or 10 mg film-coated tablets to be administered twice a day for a dosage range of 10-40 mg/day. However, lower and higher dosages can be and have been administered within the range of 5-100 mg/day and the broader range of 5-150 mg/day.

In a preferred embodiment, memantine will be administered within the range from 5 mg to 100 mg per day, preferably, from 10 to 40 mg per day, more preferably about 20 mg/day, as is specified in claims 3-5.

However, for any pharmaceutical composition used in the invention, the therapeutically effective dose can be estimated initially from animal models to achieve a circulating plasma concentration range that includes the IC₅₀ (*i*.*e*., the concentration of the test compound which achieves a half-maximal inhibition of NMDA receptor activity in the relevant areas of the brain). Dose-response curves derived from animal systems are then used to determine testing doses for the initial clinical studies in humans. In safety determinations for each composition, the dose and frequency of administration should meet or exceed those anticipated for use in the clinical trial.

Other factors to consider are the dosage procedure, the conditions of a patient or a subject animal such as age, body weight, sex, sensitivity, feed, dosage period, drugs used in combination, seriousness of the disease. The appropriate dose and dosage times under certain conditions can be determined by the test based on the above-described indices but may be refined and ultimately decided according to the judgment of the practitioner and each patient's circumstances (age, general condition, severity of symptoms, sex, etc.) according to standard clinical techniques.

Toxicity and therapeutic efficacy of the compositions of the invention can be determined by standard pharmaceutical procedures in experimental animals, *e.g*., by determining the LD₅₀ (the dose lethal to 50% of the population) and the ED₅₀ (the dose therapeutically effective in 50% of the population). The dose ratio between therapeutic and toxic effects is the therapeutic index and it can be expressed as the ratio ED₅₀/LD₅₀. Compositions that exhibit large therapeutic indices are preferred.

Treatment duration can be short-term, *e.g*., several weeks (for example 10-14 weeks), or long-term until the attending physician deems further administration no longer is necessary to obtain a benefit.

The invention is also described by means of particular examples. However, the use of such examples is illustrative only The invention is therefore to be limited only by the terms of the appended claim

### EXAMPLE 1: RANDOMIZED, PLACEBO-CONTROLLED, DOUBLE-BLIND STUDY OF MEMANTINE FOR THE TREATMENT OF MILD-TO-MODERATE ALZHEIMER'S DISEASE

The study consisted of 1-2 weeks of single-blind placebo treatment followed by 24 weeks of double-blind treatment. Results were evaluated over seven clinical visits; at initial screening, baseline, and the end of weeks 4, 8, 12, 18 and 24.

**Patient population and diagnosis.** The study population consisted of outpatients who were at least 50 years old and were diagnosed with probable AD at screening according to the NINCDS-ADRDA criteria. These criteria consist of the following:
- dementia established by clinical exam and by the MMSE and further confirmed by neurological tests
- deficits in 2 or more areas of cognition
- progressive worsening of memory and other cognitive functions
- no disturbance of conscience
- onset between the ages of 40-90
- absence of systemic diseases, such as cardiovascular diseases, that could explain the cognitive changes

### Supportive criteria include:

- progressive deterioration of specific cognitive functions such as language, motor skills, and perception (aphasia, apraxia, agnosia, respectively).
- impaired activities of daily living
- positive family history, particularly if documented neuropathologically.
- lab results: normal lumbar puncture, EEG, and evidence of cerebral atrophy on CT or MRI

### Clinical features may also include:

- plateaus in clinical course
- associated symptoms: depression, insomnia, incontinence, delusions, illusions, hallucinations, catastrophic verbal, emotional or physical outbursts, sexual disorders, and weight loss;
- increased motor tone, myoclonus or gait disorder
- seizures
- normal CT for age

These criteria also include inventories such as the Hachinski scale (Rosen et al., Ann Neurol. 1980; 7(5):486-8-ischemia, ≤4), Montgomery and Asburg Depression Rating Scale (MADRS-<22 at screening), and the MMSE described below. Confirmatory CT or MR scans were also conducted within 12 months of entry. Informed consent was obtained by the patient, caregiver or legal representative prior to initiation. A complete physical, and blood and urine samples were also obtained at screening.

The population of patients who received at least one dose of double-blind memantine, and completed at least one post-baseline efficacy evaluation of either the ADAS-cog or the CIBIC-plus is designated the "intent-to-treat" (ITT) population. The population of randomized patients who received at least one dose of double-blind study medication is designated the "safety population". The randomized population designates all patients randomized into the study.

Patient population and baseline characteristics are presented in Table 1 below:

**Table 1: Trial and Baseline Characteristics (Safety Population)**

| | | **Placebo** | **Memantine** | **Overall** |
|---|---|---|---|---|
| Population, n | | | | |
| | Randomized | 202 | 201 | 403 |
| | Safety(Treated) | 202 | 201 | 403 |
| | Intent-to-Treat | 198 | 196 | 394 |
| | | | | |
| Dropout, % | Overall | 17.30% | 17.90% | 17.60% |
| | Dropout due to AE | 5.0% | 9.50% | 7.20% |
| | | | | |
| Age (Years) | | | | |
| | Mean | 77 | 78 | 77.5 |
| | Median | 78 | 79 | 79 |
| | Range | 51 - 95 | 53 - 92 | 51 - 95 |
| | | | | |
| Baseline MMSE ≤ 22 | | | | |
| | Mean | 17.2 | 17.4 | 17.3 |
| | <=14, n | 49 | 50 | 99 |
| | > 14, n | 153 | 151 | 304 |

### Interventions

Patients were assigned to double-blind treatment for 24 weeks preceded by a 7-14 day single-blind placebo lead-in period. The lead-in period was solely for the purpose of assessing compliance. Patients were randomized to memantine or placebo groups in permuted blocks of four in accordance with the randomization list. At the baseline visit, patients were sequentially assigned the randomization numbers. No individual patient randomization code was revealed during the trial. Treatment codes were unblinded at the termination of the study after the database was locked.

This study was conducted as a fixed-dose study. All patients assigned to the memantine group received an initial dose of 5 mg per day; the dose was titrated in 5 mg weekly increments to a final dose of 20 mg/day (administered as two 5 mg tablets twice a day). Study drug was provided as blister packs containing masked study medication such that placebo and memantine tablets were visually identical, and all patients received four tablets of study medication daily (in combinations of memantine 5 mg tablets and matching placebo tablets). Transient dose adjustments were permitted between weeks 3 and 8 for patients experiencing dose-limiting adverse events: However, all patients were required to receive the target dose of 20 mg/day by the end of week 8. Patients not able to tolerate the target dose by week 8 were discontinued from the study.

No other NMDA-receptor antagonist was administered as a concomitant medication by the patient population.

**Statistical analysis.** For primary efficacy measurements, (*e.g*., change from Baseline (visit 2) to Week 24 in the total ADAS-cog scores from the ITT population) the comparison between memantine and placebo were performed using two-way analysis of covariance (ANCOVA) with treatment group and center as the two factors, and the baseline scores as covariate. Both the Last Observation Carried Forward (LOCF) imputation approach and the Observed Cases (OC) approach were performed at week 24.

The CIBIC-plus rating was analyzed using the CMH test, controlling for study center (35 total centers). Descriptive statistics were calculated by visit.

For secondary efficacy measurements, the comparison between memantine and placebo was performed using two-way analysis of covariance (ANCOVA) with treatment group and center as the two main effects, and the baseline scores as covariate. Descriptive statistics were calculated by visit. Again, all analyses were performed using both the Last Observation Carried Forward (LOCF) imputation approach and the Observed Cases (OC) approach.

Results of ANCOVA were summarized using least square (LS) means for each treatment group with corresponding standard error (SE), the between-treatment (2-24 week) difference in the least square means with corresponding 95% confidence interval, and the between-treatment p-value corresponds to the SAS Type III sum of squares.

### Results

**Efficacy.** Statistical improvements in the primary efficacy parameters of ADAS-org and CIBIC-plus were achieved by LOCF analysis in the ITT population, with a p value of 0.003 and 0.004, respectively. See Table 2 below:

**Table 2: Efficacy at Week 24 (ITT Population)**

| | **LOCF Analysis** | | **OC Analysis** | |
|---|---|---|---|---|
| | **Difference*** | **p-value** | **Difference*** | **p-value** |
| ADAS-cog | -1.9 | 0.003 | -1.1 | 0.130 |
| CIBIC+ | -0.32 | 0.004 | -0.26 | 0.030 |
| ADCS-ADL | 0.1 | 0.890 | 0.0 | 0.975 |
| NPI total | -3.5 | 0.011 | -2.1 | 0.143 |

| | | | | |
|---|---|---|---|---|
| *: memantine minus placebo, based on mean value for CIBIC+, and LS Mean value for ADAS-cog, ADL, and NPI-total | | | | |

Notably, as determined by CIBIC-plus and LOCF analysis in the ITT population, of 196 patients randomized to memantine, 9 demonstrated "marked improvement" and 27 demonstrated "moderate improvement compared with 7 and 21 in the population randomized to placebo (198). Similarly, 18 and 1 of the memantine-treated population, demonstrated moderate worsening or marked worsening, respectively, compared with 34 and 3, respectively, of the placebo-treated group (data not shown). By OC analysis, 8 memantine-treated patients exhibited moderate improvement and 24 exhibited minimal improvement, compared with 7 and 19 patients, respectively, in the placebo-treated population. In addition, 1 memantine-treated patient demonstrated marked worsening compared to 3 in the placebo group, and 17 memantine-treated patients compared with 27 placebo-treated patients showed moderate worsening (data not shown).

The population of CIBIC-plus and ADAS-cog responders was compared to that of the non-responders to evaluate the placebo effect. As shown in Table 3, below, CIBIC-plus analysis according to LOCF demonstrated that 100 patients who were designated responders received the placebo, while 132 received memantine, and 97 reported "non-responders" received placebo, while only 64 non-responders received memantine. Similar results with OC analysis were obtained from CIBIC-plus, where 85 "responders" received placebo and 108 received memantine, and 81 "non-responders received placebo and 56 received memantine.

**Table 3: CIBIC+ and ADAS-cog Responders at Week 24 (ITT Population)**

| | | **LOCF Analysis** | | **OC Analaysis** | |
|---|---|---|---|---|---|
| | | **Pla/Mem** | **p-value** | **Pla/Mem** | **p-value** |
| CIBIC+ | | | | | |
| | Responder | 100/132 | 0.001 | 85/108 | 0.007 |
| | Non-Responder | 97/64 | | 81/56 | |
| ADAS-cog | | | | | |
| | Responder | 40/47 | 0.352 | 33/34 | 0.846 |
| | Non-Responder | 158/148 | | 129/126 | |

| | | | | | |
|---|---|---|---|---|---|
| Note: Responder in ADAS-cog: change from baseline in total ADAS-cog <= -4 Responder in CIBIC+: CIBIC+ ratings = 1, 2, 3, 4 (no change or improvement) | | | | | |

Of the safety patient population, about 66% had previously taken at least one medication prior to this study (no patients had been administered any anti-dementia medication for one month prior to commencement of this study). Of those, about 69% of the placebo-treated and 62% of the memantine-treated patients had been on previous dementia medication. Of those, about 65% of the placebo-treated and 59% of the memantine-treated individuals had used an AChEI such as donepezil, galantamine, or rivastigmine, These results show memantine was effective for individuals with mild-to-moderate SDAT in whom prior treatment with other anti-dementia drugs, notably AChEIs has been discontinued.

Results were shown as early as week 8, by LOCF, when the LS mean change of the placebo-randomized group, measured using ADAS-cog, was 0.2 and that of the memantine-randomized group was -1.2 (p value of 0.003). By week 12, the LS mean was -0.1 for the placebo-treated group and -1.4 for the memantine-treated group (p = 0.009).

OC measurements of ADAS-cog also showed significant improvement by week 8 in the memantine-treated population, where the LS mean was -1.1 compared to that of 0.2 for the placebo (p = 0.006).

Similarly for CIBIC-plus, statistically significant improvement in the memantine-treated group compared with the placebo was achieved by week 12 using LOCF and OC.

For NPI Total, significance using LOCF was achieved by week 24. Of the individual NPI criteria, three were significantly better in the memantine-treated compared with the placebo-treated group. Achievement of statistical significance in total NPI score is attributed to a sub-threshold benefit in several criteria, not only to the three criteria that showed significant change individually.

The failure to observe a statistically significant change in ACDS-ADL may have been due to the particular selection of the 23 questions or simply to the fact that this type of patient population (mild-to-moderate AD) does not yet display severe deterioration in the various symptoms that make up the evaluation criteria. In any event, a prior failure of a clinical study to show that memantine conferred any additional benefit over donepezil in patients with mild-to-moderate AD (especially after success of a similar study in moderate-to-severe AD patients) would have discouraged those skilled in the art from using memantine in the mild-to-moderate population.

Each of the foregoing considerations and all of them make the present clinical results unexpected.

### EXAMPLE 2: THE EFFECTS OF MEMANTINE ON BRAIN GLUCOSE METABOLISM IN MILD-TO-MODERATE ALZHEIMER'S DISEASE

### Methods

***Drug treatment*.** As stated above, patients were randomized to receive 20 mg/day (10 b.i.d.) memantine or placebo for 24 weeks. PET scans were performed for 10 (5 receiving memantine and 5 receiving placebo) patients at baseline and at the end of week 24.

***PET Scan Acquisition***. Subjects were PET scanned at the University of California, Irvine Brain Imaging Center using a GE 2048-15B dedicated head scanner. Thirty slices at 6.5-mm intervals (15 in each of two sets) were obtained so as to cover the entire brain. Attenuation correction was performed by obtaining a transmission scan using a ⁶⁸Ge pin source. A thermosetting plastic facemask was used to hold the subject stationary during the 60 minutes of total image acquisition. Scans were reconstructed with a blank and a transmission scan. PET image count was used for study analyses and adjusted for intrasubject global differences using normalization (proportional scaling).

***PET Image Methodology and Analysis***. Differences in regional FDG uptake were analyzed using SPM99 software (Wellcome Department of Cognitive Neurology, London, UK), implemented on the Matlab platform (MathWorks Inc., Sherbom, MA). Statistical parametric maps (SPMs) are spatially extended statistical processes that are constructed to test hypotheses about regionally specific effects in neuroimaging data. Statistical parametric mapping combines the general linear model and the theory of Gaussian random fields to make statistical inferences about regional effects. The images were spatially normalized using a twelve parameter affine transformation, estimated by minimizing the residual sum of squares between each scan, and a reference or template image conforming to the standard space defined by Talairach and Tournoux. The original image matrix obtained at 256 x 256 x 30 with a voxel size of 4.5 mm x 4.5 mm x 6.5 mm plane separation where transformed and resliced to a 79 x95 x 68 matrix with a voxel size of 2 mm x 2 mm x 2 mm plane separation corresponding more closely to the Talairach standard anatomical space, and were smoothed using an (5 mm FWHM) isotropic Gaussian kernel. Prior to statistical inference, images were scaled to a canonical normal to account for global variations between subjects over time (proportional scaling).

To test the hypotheses regarding regionally specific condition effects, the estimates were compared using linear contrasts. The resulting set of voxel values for each contrast represents a parametric mapping of the t-statistics, SPM(t), which were transformed into the unit normal distribution SPM(z) and thresholded at p < .025 uncorrected. The resulting foci were then characterized in terms of peak height (*u*). The significance of each region was estimated based on the probability that the observed peak height could have occurred by chance P(Zₘₐₓ > *u*). Because the maximum intensity and the spatial extent of a region above *u* are not independent, a simple "Bonferroni" correction for multiple comparisons would be inappropriate. Accordingly, spatial extent thresholding based on the Gaussian random field theory was performed at 10 voxels to correct for multiple comparisons.

Anatomical localization of significant regions was accomplished through comparison with the Talaraich and Tournoux atlas and confirmation by a neuroanatomist who was blind to group assignments. The Talairach and Tournoux coordinates are provided in parentheses following the relevant brain regions. It should be noted, however, that present PET imaging technology does not allow for precise anatomical localizations to specific subcortical nuclei and sublobular cortical areas. Some approaches have applied co-registration within individual MRI templates in order to aid in verifying anatomical localization. Such templates were not available in this study. The interpretation by blinded neuroanatomist aided in anatomical verification by employing dynamic interpretation of brain circuits based on known anatomical structures and following changes in regional metabolism between adjacent slices.

***Snodgrass Picture Naming Task***. Several studies of semantic abilities in dementia of the Alzheimer Type suggest that their semantic abnormalities may affect certain categories of knowledge. In the present invention, all patients who were subjected to PET scanning were evaluated during imaging using the Snodgrass picture naming activation task, The use of a task during the FDG uptake was used to image increases in the hippocampus and temporal lobe. This Snodgrass Picture Naming activation task was sensitive to object naming, which is impaired in mild to moderate AD. The Snodgrass picture naming activation task and performance analysis was based, in part, on the speed of identification and articulation of the name that corresponds to a set of 260 pictures (Snodgrass and Vanderwart, J. Experimental Psychology: Human Learning and Memory 1980; 6(2): 174-215). The pictures are black-and-white line drawings executed according to a set of rules that provide consistency of pictorial representation. The pictures were standardized on four variables of central relevance to memory and cognitive processing:

### Results

In the placebo vs. baseline condition (**Figure 1**), most of the changes are decreases in GMR bilaterally throughout cortical and subcortical regions. These include bilateral decreases in orbital cortex, posterior cingulate, retrosplenial, and posterior parahippocampal gyrus, superior temporal gyrus, visual cortices, dorsolateral prefrontal cortex, posterior thalamus, and cerebellar cortex. Unilateral decreases were observed in the right temporal pole and middle temporal gyrus, right medial thalamus, left putamen, right superior parietal and right premotor cortex, left motor cortex, and right insula. Limited increases were demonstrated in limbic structures such as the lateral amygdala, orbital cortex, and the border between the parahippocampal gyrus and medial fusiform gyrus, and anterior temporal lobe.

In the memantine-treated patients (**Figure 2**), metabolic increases were observed bilaterally throughout the CNS, including the cerebral cortex, cerebellum, and brainstem. In the cerebral cortex, greater increases are found in the right hemisphere, as well as bilaterally in the orbital cortex, visual cortices, superior and inferior parietal cortex, posterior cingulate, retrosplenial cortex and posterior parahippocampal gyrus, medial prefrontal and anterior cingulate cortices, premotor cortex, and dorsal prefrontal cortex. Subcortically, increases were present over a broad region of the left pontine nuclei, the right tectum, left subthalamic nucleus/red nucleus region, and right putamen. An isolated region of the left visual cortex and left putamen exhibited decreases in glucose metabolism.

Regarding the Snodgrass picture naming activation task, memantine-treated subjects were able to perform the task at better than chance. There was no significant difference in Snodgrass performance between the two treatment groups.

### Discussion

In this pilot study, it was shown that the placebo-treated patients generally showed expected metabolic decreases in most cortical areas over the 24-week period. There were also mild metabolic increases in some limbic areas (*e*.*g*., orbital cortex and anterior temporal lobe structures, as has previously shown (Potkin et al., Int J Neuropsychopharmacol. 2001; 4(3):223-30). In the medial occipital cortex, some metabolic decreases were also observed in the placebo-treated patients in the present study. This is in contrast to mild metabolic increases in the occipital pole observed in our previous study. The metabolic changes observed in the placebo-treated patients were consistent with decreased functioning of the attentional system, especially the posterior attentional system (tectum, posterior thalamus, posterior cingulate, and superior parietal lobule and precuneus).

An entirely different pattern was observed in the memantine-treated subjects. In general, the areas that showed statistically significant decreases in the placebo-treated patients showed metabolic increase. These areas of increased metabolism following memantine include cortical structures of the anterior and posterior attentional system, as well as premotor, inferior parietal (language-related), temporal tip, and orbital and dorsal prefrontal cortices. In addition, isolated increases were found unilaterally in the tectum as well as basal ganglia/extrapyramidal structures (pontine nuclei, subthalamic/red nucleus region, putamen, and cerebellum).

The use of PET to document the effects of treatment has distinct advantages in being able to examine regional changes in brain metabolism over time. These measures offer advantages over current diagnostic measures, *e.g*. CIBC-Plus and ADAS-cog. For example, the CIBIC-Plus is dependent on caregiver observations, which may be affected by fluctuating caregiver capacity. FDG PET overcomes some of these limitations.

In addition, all subjects were able to perform the Snodgrass Picture Naming activation task at better than chance; therefore, the task did not suffer from either floor or ceiling effects, unlike many memory tasks. This feature of the test procedure guards against motivation apathy and discouragement as factors interfering with performance. There was no significant difference in Snodgrass performance; therefore, performance does not explain the observed findings.

Memantine, a noncompetitive NMDA antagonist with demonstrated efficacy in patients with moderate to severe AD, was also effective for the treatment of mild-to-moderate AD, and can reverse the expected metabolic changes associated with regression in AD.

## Claims

1. Use of memantine or a pharmaceutically acceptable salt thereof in the manufacture of a medicament for monotherapeutically treating mild-to-moderate Alzheimer's disease, wherein the medicament is manufactured for administration of memantine at an initial dose of 5 mg/day rising weekly by 5 mg/day to a final dose of 20 mg/day.

2. Use of memantine or a pharmaceutically acceptable salt thereof in the manufacture of a medicament for monotherapeutically treating mild-to-moderate Alzheimer's disease, wherein the medicament is manufactured for administration to subjects with mild-to-moderate Alzheimer's disease who have previously been treated with an acetylcholinesterase inhibitor but have discontinued said previous treatment at least one day prior to commencement of memantine administration.

3. Use as claimed in claim 2 wherein the medicament is manufactured for administration of memantine at a dose in the range 5 to 100 mg/day.

4. Use as claimed in claim 3 wherein said dose is in the range 10 to 40 mg/day.

5. Use as claimed in claim 4 wherein said dose is about 20 mg/day.

6. Use as claimed in claim 2 wherein the medicament is manufactured for administration of memantine at an initial dose of 5 mg/day rising weekly by 5 mg/day to a final dose of 20 mg/day.

## Patentansprüche

1. Verwendung von Memantin oder einem pharmazeutisch annehmbaren Salz davon zur Herstellung eines Medikaments für die monotherapeutische Behandlung einer leichten bis moderaten Alzheimer Krankheit, wobei das Medikament zur Verabreichung von Memantin in einer anfänglichen Dosis von 5 mg/Tag wöchentlich ansteigend um 5 mg/Tag bis zu einer Enddosis von 20 mg/Tag hergestellt ist.

2. Verwendung von Memantin oder einem pharmazeutisch annehmbaren Salz davon zur Herstellung eines Medikaments für die monotherapeutische Behandlung einer leichten bis moderaten Alzheimer Krankheit, wobei das Medikament zur Verabreichung an Patienten mit leichter bis moderater Alzheimer Krankheit, die vorher mit einem Acetylcholinesterase-Inhibitor behandelt worden sind aber diese vorherige Behandlung mindestens einen Tag vor dem Beginn der Memantinverabreichung abgebrochen haben, hergestellt ist.

3. Verwendung wie beansprucht in Anspruch 2, wobei das Medikament zur Verabreichung von Memantin in einer Dosis im Bereich von 5 bis 100 mg/Tag hergestellt ist.

4. Verwendung wie beansprucht in Anspruch 3, wobei diese Dosis im Bereich von 10 bis 40 mg/Tag ist.

5. Verwendung wie beansprucht in Anspruch 4, wobei diese Dosis etwa 20 mg/Tag ist.

6. Verwendung wie beansprucht in Anspruch 2, wobei das Medikament zur Verabreichung von Memantin in einer anfänglichen Dosis von 5 mg/Tag wöchentlich ansteigend um 5 mg/Tag bis zu einer Enddosis von 20 mg/Tag hergestellt ist.

## Revendications

1. Utilisation de la mémantine ou d'un sel pharmaceutiquement acceptable de celle-ci dans la fabrication d'un médicament pour traiter monothérapeutiquement la maladie d'Alzheimer légère à modérée, dans laquelle le médicament est fabriqué pour une administration de la mémantine à une dose initiale de 5mg/jour augmentant chaque semaine de 5mg/jour jusqu'à la dose finale de 20mg/jour.

2. Utilisation de la mémantine ou d'un sel pharmaceutiquement acceptable de celle-ci dans la fabrication d'un médicament pour traiter monothérapeutiquement la maladie d'Alzheimer légère à modérée, dans laquelle le médicament est fabriqué pour une administration à des sujets avec une maladie d'Alzheimer légère à modérée qui ont été traités auparavant avec un inhibiteur de l'acétylcholine mais ont arrêté ledit traitement antérieur au moins un jour avant de commencer l'administration de mémantine.

3. Utilisation telle que revendiquée dans la revendication 2 dans laquelle le médicament est fabriqué pour une administration de mémantine à une dose comprise dans la gamme de 5 à 100mg/jour.

4. Utilisation telle que revendiquée dans la revendication 3 dans laquelle ladite dose est comprise dans la gamme de 10 à 40mg/jour.

5. Utilisation telle que revendiquée dans la revendication 4 dans laquelle ladite dose est d'environ 20mg/jour.

6. Utilisation telle que revendiquée dans la revendication 2 dans laquelle le médicament est fabriqué pour une administration de mémantine à une dose initiale de 5mg/jour augmentant chaque semaine de 5mg/jour jusqu'à la dose finale de 20mg/jour.
